# EUROPEAN PATENT APPLICATION

(11) **EP 4 697 427 A1**
(43) Date of publication of application: **18.02.2026**
(21) Application number: 24877445.7
(22) Date of filing: 27.09.2024
(51) Int. Cl.: H01M 10/0567, C07C 233/13, H01M 4/48, H01M 4/587, H01M 4/38, H01M 10/052

(54) **ELECTROLYTE ADDITIVE, NON-AQUEOUS ELECTROLYTE FOR LITHIUM SECONDARY BATTERY COMPRISING SAME, AND LITHIUM SECONDARY BATTERY**

(30) Priority: 12.10.2023 KR 20230136309
(71) Applicant: LG Energy Solution, Ltd., Seoul 07335 (KR)
(72) Inventor: LEE, Jae Won, Daejeon 34122 (KR); PARK, Sung Guk, Daejeon 34122 (KR); LEE, Chul Haeng, Daejeon 34122 (KR); LEE, Jung Hoon, Daejeon 34122 (KR)
(74) Representative: Hoffmann Eitle
(86) International application number: PCT/KR2024/014785
(87) International publication number: WO 2025/079907

(57) **Abstract**

The present disclosure provides an electrolyte additive, a non-aqueous electrolyte for a lithium secondary battery including the same, and a lithium secondary battery. Specifically, the present disclosure can provide a non-aqueous electrolyte for a lithium secondary battery capable of securing excellent cycle characteristics and a lithium secondary battery comprising the same by applying a non-aqueous electrolyte for a lithium secondary battery that includes an electrolyte additive capable of forming a robust SEI layer on the surface of the negative electrode.

## Description

### [TECHNICAL FIELD]

### Cross-reference to Related Applications

This application claims priority from Korean Patent Application No. 10-2023-0136309, filed on October 12, 2023, the disclosure of which is incorporated by reference herein.

### [TECHNICAL FIELD]

The present disclosure relates to an electrolyte additive, a non-aqueous electrolyte for a lithium secondary battery including the same, and a lithium secondary battery.

### [BACKGROUND ART]

As dependence on electric energy is increasing in modern society, development of a large-capacity power storage device that can stably supply power and increase production is in the spotlight.

Lithium-ion battery is a power storage device with the highest energy density among power storage devices that has been commercialized, and thus has been applied to various fields such as small electronic products, electric vehicles (EV) and power storage devices.

In particular, high output characteristics are required for lithium ion secondary batteries applied to electric vehicles, while maintaining cycle characteristics and performance in various environments. Accordingly, research is actively being conducted to develop positive and negative electrode active materials with a high theoretical capacity to improve the energy density of lithium secondary batteries. Specifically, research is being conducted to apply silicon-based negative electrode active materials such as Si or SiOₓ (0<x<2) which have a higher theoretical capacity than graphite, which is currently used in commercial batteries.

However, silicon-based negative electrode active materials have the disadvantage of experiencing rapid volume changes and expansion due to changes in the crystal structure caused by ion intercalation and deintercalation during repeated electrochemical charging and discharging or high-temperature storage, which in turn causes film fragmentation and deterioration, thereby aggravating battery degradation. In particular, the solid electrolyte interface layer (hereinafter referred to as "SEI layer") formed on the electrode surface as the carbonate-based non-aqueous solvent decomposes has weak elasticity, making it difficult to maintain the film upon the volume expansion of the silicon-based negative electrode active material, which causes battery degradation.

Therefore, there is a need to develop an electrolyte capable of solving the problem of deterioration due to the volume expansion of the silicon-based negative electrode active material by forming a stable SEI layer that can withstand the volume changes of the silicon-based negative electrode active material on the negative electrode surface.

### [DISCLOSURE OF THE INVENTION]

### [TECHNICAL PROBLEM]

The present disclosure aims to address the above problems, and provides an electrolyte additive capable of forming a robust film on the surface of a silicon-based negative electrode, and a non-aqueous electrolyte for a lithium secondary battery comprising the same.

In addition, the present disclosure provides a lithium secondary battery with improved cycle properties by including the above non-aqueous electrolyte for a lithium secondary battery.

### [TECHNICAL SOLUTION]

[1] The present disclosure provides an electrolyte additive including a compound represented by the following Formula 1: 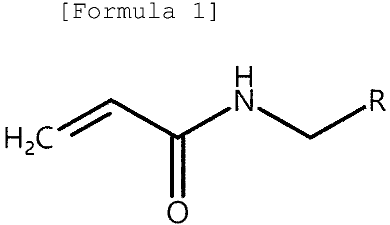
   In Formula 1 above,
   R is an alkyl group having 1 to 7 carbon atoms substituted with at least one fluorine.
[2] The present disclosure provides an electrolyte additive where in the above [1], R is an alkyl group having 1 to 5 carbon atoms substituted with at least one fluorine.
[3] The present disclosure provides an electrolyte additive where in the above [1] or [2], R is an alkyl group having 1 to 3 carbon atoms substituted with at least one fluorine in Formula 1 above.
[4]The present disclosure provides an electrolyte additive, wherein in at least one of the above [1] to [3], the compound represented by Formula 1 above is at least one of compounds represented by the following Formula 1A-1 to 1A-3: 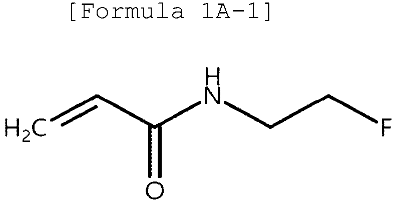 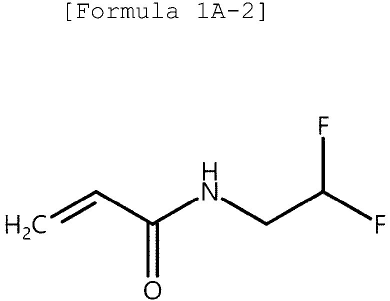 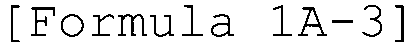 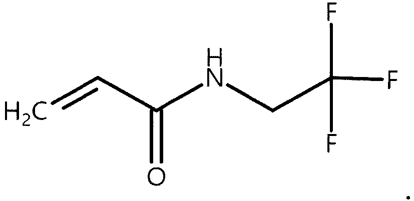
[5] The present disclosure provides a non-aqueous electrolyte for a lithium secondary battery including an electrolyte additive according to at least one of the above [1] to [4].
[6] The present disclosure provides a non-aqueous electrolyte for a lithium secondary battery, wherein in the above [5], the above electrolyte additive is present in an amount of 0.1 wt% to 10.0 wt% in the non-aqueous electrolyte for a lithium secondary battery.
[7] The present disclosure provides a non-aqueous electrolyte for a lithium secondary battery, wherein in the above [5] or [6], the non-aqueous electrolyte for a lithium secondary battery further includes a lithium salt and a non-aqueous organic solvent.
[8]The present disclosure provides a non-aqueous electrolyte for a lithium secondary battery, wherein in at least one of the above [5] to [7], the non-aqueous electrolyte for a lithium secondary battery further includes at least one additional additive selected from a cyclic carbonate-based compound, a halogen-substituted carbonate-based compound, a sultone-based compound, a sulfate-based compound, a phosphate-based or phosphite-based compound, a borate-based compound, a benzene-based compound, an amine-based compound, an imidazole-based compound, a silane-based compound, or a lithium salt-based compound.
[9] The present disclosure provides a lithium secondary battery which includes a positive electrode, a negative electrode facing the positive electrode, a separator disposed between the positive electrode and the negative electrode, and a non-aqueous electrolyte for a lithium secondary battery according to at least one of the above [5] to [8].
[10] The present disclosure provides a lithium secondary battery wherein, in the above [9], the negative electrode includes a silicon-based negative electrode active material.
[11] The present disclosure provides a lithium secondary battery wherein, in the above [9] or [10], the negative electrode further includes a carbon-based negative electrode active material.

### [ADVANTAGEOUS EFFECTS]

The non-aqueous electrolyte for a lithium secondary battery according to the present disclosure includes an electrolyte additive containing a vinyl group and nitrogen (N) atom capable of forming a robust SEI layer on the electrode surface in the structure, thereby forming a durable amide group and Li₃N-containing film on the negative electrode surface. Therefore, when the non-aqueous electrolyte for a lithium secondary battery of the present disclosure is used, a lithium secondary battery with improved cycle properties can be achieved. The non-aqueous electrolyte of the present disclosure is particularly suitable for high-power batteries using high-capacity active materials, such as high nickel-based positive electrode active materials or silicon-based negative electrode materials with significant volume expansion during charging and discharging, together.

### [MODE FOR CARRYING OUT THE INVENTION]

The terms used in the present specification and the claims are used only to describe exemplary embodiments, and are not intended to limit the present disclosure.

For example, it should be appreciated that the terms such as "include," "comprise," and "have" are intended to embody specific features, numbers, steps, elements, or combinations thereof, and allow other components to be added unless the terms are used with the terms only.

Also, in the present specification, the expression "%" denotes wt% unless explicitly stated otherwise.

Unless otherwise defined in the specification, the expression "substitution" denotes that at least one hydrogen bonded to carbon is substituted with an element other than hydrogen, and for example, it means being substituted with an alkyl group having 1 to 5 carbon atoms or a fluorine element.

Among conventional negative electrode materials for lithium ion batteries, silicon-based negative electrode active materials have the advantage of excellent capacity per weight. However, the intercalation and deintercalation of lithium ions caused by repeated charging and discharging can lead to serious volume expansion (over 300%) and shrinkage, causing cracks or damaging the SEI layer formed on the surface of the silicon-based negative electrode active material. These problems lead to side reactions between the surface of the negative electrode active material and the electrolyte, forming a thick and unstable film at the silicon-based negative electrode active material and the electrolyte interface, increasing resistance, and increasing the consumption of the lithium ion source. As a result, cycle characteristics and charge/discharge characteristics deteriorate.

To solve the above problem, the present inventors aim to provide an electrolyte additive capable of forming a robust SEI film on the silicon-based negative electrode and a non-aqueous electrolyte including the same, and aim to provide a lithium secondary battery with improved high-rate charging/discharging properties at high temperatures by using the same.

Hereinafter, the present disclosure will be described in more detail.

The electrolyte additive according to the present disclosure, a non-aqueous electrolyte for a lithium secondary battery including the same, and a lithium secondary battery may include at least one of the constitutions disclosed below, and may include any combination between technically possible constitutions among the following constitutions.

### Electrolyte Additive

Specifically, the present disclosure provides an electrolyte additive including a compound represented by the following Formula 1. 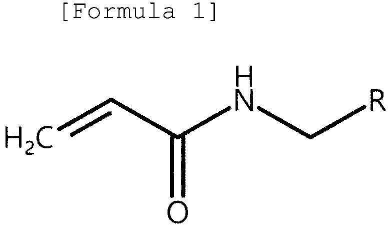
In Formula 1,
R is an alkyl group having 1 to 7 carbon atoms substituted with at least one fluorine.

The compound represented by Formula 1 included as the above electrolyte additive includes a vinyl group and a nitrogen element in its structure, so that it is reductively decomposed before the organic solvent during charging and discharging, thereby forming a durable amide group and a partially Li₃N-containing film on the surface of the silicon-based negative electrode. As a result, SEI film cracking caused by the shrinkage/expansion of the silicon-based negative electrode active material can be suppressed. In addition, as the compound represented by Formula 1 contains a fluorine element in its structure, it can form a fluorine-containing film with strong high-temperature durability and thermal stability on the negative electrode surface to minimize the decomposition of the SEI layer at high temperatures, thereby allowing for reduction in the side reactions caused by direct contact between the silicon and the electrolyte. In particular, since the compound represented by Formula 1 of the present disclosure contains an amide group at the center of its structure, it exhibits excellent film stability compared to compounds represented by [Formula i] or [Formula ii] below, which contain a carboxylate group in its their structure. This not only delays film decomposition and further electrolyte decomposition reactions, but the amount decomposed in the form of CO₂ or hydrocarbon gases such as CₓH_{y} (where x is 1 to 5, y is 3 to 12) is low upon decomposition of the film, so that deterioration caused by gas inside the battery and venting can be delayed, thereby allowing for further improvement of the SEI layer stability. Therefore, a lithium secondary battery with improved cycle and capacity properties can be manufactured with the non-aqueous electrolyte for a lithium secondary battery comprising such an electrolyte additive.

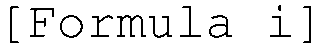

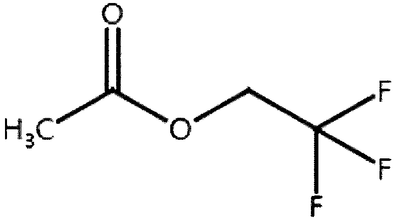

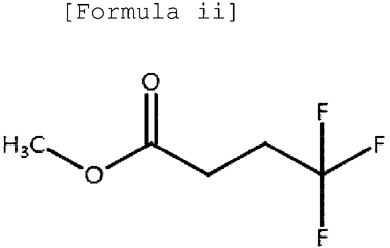

Meanwhile, in Formula 1, R may be an alkyl group having 1 to 5 carbon atoms substituted with at least one fluorine. More specifically, R may be an alkyl group having 1 to 3 carbon atoms substituted with at least one fluorine.

More specifically, the compound represented by Formula 1 above may be at least one of compounds represented by Formula 1A-1 to Formula 1A-3 below. Preferably, the compound represented by Formula 1 above may be a compound represented by the following Formula 1A-3, in which R, which is relatively highly substituted with highly durable fluorine, has a perfluoro structure.

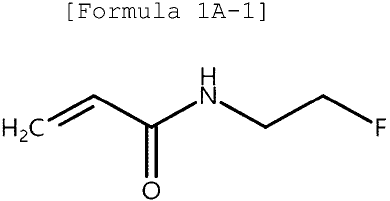

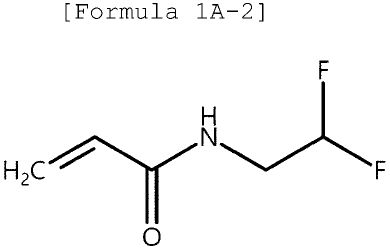

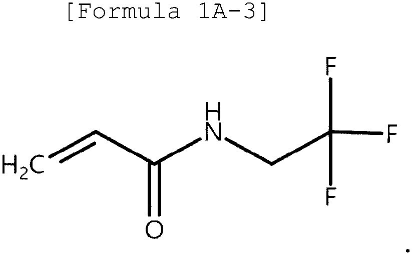

### Non-aqueous electrolyte for a lithium secondary battery

Further, in an embodiment, the present disclosure provides a non-aqueous electrolyte for a lithium secondary battery comprising the electrolyte additive of the present disclosure.

The non-aqueous electrolyte for a lithium secondary battery may further comprise a lithium salt, an organic solvent and optionally an additional additive.

### (1) Electrolyte Additive

The non-aqueous electrolyte for a lithium secondary battery of the present disclosure may include an electrolyte additive including the compound represented by the above Formula 1. Since descriptions of the above compound overlap with those described above, the descriptions thereof will be omitted.

Meanwhile, the electrolyte additive may be present in an amount of 10.0 wt% or less, specifically 0.1 wt% to 10.0 wt% based on the total weight of the non-aqueous electrolyte, considering the effect of forming a stable film on the electrode surface and effect of scavenging the thermal decomposition products of lithium salts.

If the above electrolyte additive is included within the above content range, it may effectively suppress the elution of the transition metals of the positive electrode active materials at high temperatures by forming a robust film on the positive electrode surface and effectively scavenge the thermal decomposition products of lithium salts, while suppressing the disadvantages such as side reactions, capacity deterioration and increased resistance caused by additives as much as possible, thereby achieving excellent high-temperature durability.

If the content of the electrolyte additive is 0.1 wt% or more, the effect of scavenging the thermal decomposition products of lithium salts can be maintained even if the operation time is increased, and the effect of suppressing the elution of the transition metals can be further improved by forming a stable film on the electrode surfaces. In addition, if the content of the electrolyte additive is 10.0 wt% or less, side reactions caused by additives included in a slightly large amount can be prevented.

Specifically, the electrolyte additive may be present in an amount of 0.1 wt% to 8.0 wt% or 0.5 wt% to 8.0 wt%, more specifically 0.5 wt% to 5.0 wt%, and even more specifically 0.5 wt% to 3.0 wt% based on a total weight of the non-aqueous electrolyte for a lithium secondary battery.

### (2) Lithium Salt

Any lithium salt typically used in a non-aqueous electrolyte for a lithium secondary battery may be used without limitation as the lithium salt, and for example, the lithium salt may include Li⁺ as a cation, and may include at least one selected from F⁻, Cl⁻, Br⁻, I⁻, NO₃⁻, N(CN)₂⁻, BF₄⁻, ClO₄⁻, B₁₀Cl₁₀⁻, AlCl₄⁻, AlO₄⁻, PF₆⁻, CF₃SO₃⁻, CH₃CO₂⁻, CF₃CO₂⁻, AsF₆⁻, SbF₆⁻, CH₃SO₃⁻, (CF₃CF₂SO₂)₂N⁻, (CF₃SO₂)₂N⁻, (FSO₂)₂N⁻, BF₂C₂O₄⁻, BC₄O₈⁻, PF₄C₂O₄⁻, PF₂C₄O₈⁻, (CF₃)₂PF₄⁻, (CF₃)₃PF₃⁻, (CF₃)₄PF₂⁻, (CF₃)₅PF⁻, (CF₃)₆P⁻, C₄F₉SO₃⁻, CF₃CF₂SO₃⁻, CF₃CF₂(CF₃)₂CO⁻, (CF₃SO₂)₂CH⁻ or CF₃(CF₂)₇SO₃⁻ as a anion.

Specifically, the lithium salt may include a single material selected from LiCl, LiBr, LiI, LiBF₄, LiClO₄, LiB₁₀Cl₁₀, LiAlCl₄, LiAlO₄, LiPF₆, LiCF₃SO₃, LiCH₃CO₂, LiCF₃CO₂, LiAsF₆, LiSbF₆, LiCH₃SO₃, LiN(SO₂F)₂ (Lithium bis(fluorosulfonyl)imide, LiFSI), LiN(SO₂CF₂CF₃)₂ (lithium bis(pentafluoroethanesulfonyl)imide, LiBETI) or LiN(SO₂CF₃)₂ (lithium bis(trifluoromethane sulfonyl)imide, LiTFSI), or a mixture of two or more thereof. In addition to these, lithium salts typically used in an electrolyte for a lithium secondary battery may be used without limitation.

The above lithium salt may be appropriately changed within a generally usable range, but may be included in the electrolyte at a concentration of 0.8M to 4.0M, and specifically, 1.0M to 3.0M, to obtain an optimal effect of forming a film for preventing the electrode surface from being corroded.

When the concentration of the lithium salt is within the above range, the viscosity of the non-aqueous electrolyte may be controlled to achieve optimal impregnation, and the mobility of lithium ions may be improved, thereby obtaining the effect of improving the capacity characteristics and cycle characteristics of a lithium secondary battery.

### (3) Non-aqueous organic solvent

In addition, a non-aqueous organic solvent will be explained as follows.

Various organic solvents typically used in a non-aqueous electrolyte may be used as the non-aqueous organic solvent without limitation, and it is not limited as long as it may minimize the decomposition due to an oxidation reaction or the like during the charging and discharging of the secondary battery and may exhibit desired characteristics with the additive.

Specifically, the non-aqueous organic solvent may include (i) a cyclic carbonate-based organic solvent, (ii) a linear carbonate-based organic solvent, or (iii) a mixture thereof.

The (i) cyclic carbonate-based organic solvent is an organic solvent having high viscosity and is an organic solvent capable of dissociating a lithium salt well in an electrolyte due to its high dielectric constant, and specific examples thereof may include at least one organic solvent selected from ethylene carbonate (EC), propylene carbonate (PC), 1,2-butylene carbonate, 2,3-butylene carbonate, 1,2-pentylene carbonate, 2,3-pentylene carbonate, or vinylene carbonate, and specifically may include at least one of ethylene carbonate or propylene carbonate.

The (ii) linear carbonate-based organic solvent is an organic solvent having low viscosity and low permittivity, wherein typical examples of the linear carbonate-based organic solvent may include at least one organic solvent selected from dimethyl carbonate (DMC), diethyl carbonate (DEC), dipropyl carbonate, ethyl methyl carbonate (EMC), methylpropyl carbonate, orethylpropyl carbonate, and specifically it may include at least one of dimethyl carbonate or ethyl methyl carbonate.

The non-aqueous electrolyte for a lithium secondary battery according to the present disclosure may use a mixture of the (i) cyclic carbonate-based organic solvent and the (ii) linear carbonate-based organic solvent to secure a high ionic conductivity, wherein the cyclic carbonate-based organic solvent or the linear carbonate-based organic solvent may be mixed in a volume ratio of 10:90 to 50:50, specifically 20:80 to 40:60.

In addition, the non-aqueous electrolyte for a lithium secondary battery of the present disclosure may additionally include, as the non-aqueous organic solvent, at least one ester-based organic solvent of (iv) a linear ester-based organic solvent and (v) a cyclic ester-based organic solvent having a lower melting point and higher stability at high temperatures compared to the cyclic carbonate-based organic solvent and/or the linear carbonate-based organic solvent.

Typical examples of the (iv) linear ester-based organic solvent may be at least one organic solvent selected from methyl acetate, ethyl acetate, propyl acetate, methyl propionate, ethyl propionate, propyl propionate, or butyl propionate, and specifically may include at least one of ethyl propionate or propyl propionate.

The (v) cyclic ester-based organic solvent may include at least one organic solvent selected from γ-butyrolactone, γ-valerolactone, γ-caprolactone, σ-valerolactone, or ε-caprolactone.

Meanwhile, the remainder excluding the lithium salt, electrolyte additive and additional additive as described below in the non-aqueous electrolyte for a lithium secondary battery may all be non-aqueous organic solvents unless otherwise stated.

### (4) Additional Additive

In addition, in order to prevent an electrolyte from being decomposed to cause collapse of a negative electrode in a high output environment, or further improve low-temperature high-rate discharge characteristics, high-temperature stability, overcharge protection, and a battery swelling suppression effect at high temperatures, the non-aqueous electrolyte for a lithium secondary battery according to the present disclosure may further include other additional additives in the above electrolyte, if necessary. Meanwhile, when other additives are included, the compound represented by Formula 1 above may be referred to as the first additive, and the other additive may be referred to as the second additive.

Examples of the additional additive may be at least one selected from a cyclic carbonate-based compound, a halogen-substituted carbonate-based compound, a sultone-based compound, a sulfate-based compound, a phosphate-based compound, a borate-based compound, a nitrile-based compound, a benzene-based compound, an amine-based compound, a silane-based compound, or a lithium salt-based compound.

The cyclic carbonate-based compound may include vinylene carbonate (VC) or vinyl ethylene carbonate.

The halogen-substituted carbonate-based compound may include fluoroethylene carbonate (FEC).

The sultone-based compound may include at least one compound selected from 1,3-propane sultone (PS), 1,4-butane sultone, ethane sultone, 1,3-propene sultone (PRS), 1,4-butene sultone, or 1-methyl-1,3-propene sultone.

The sulfate-based compound may include ethylene sulfate (Esa), trimethylene sulfate (TMS), or methyl trimethylene sulfate (MTMS).

The phosphate-based compound may include one or more compounds selected from lithium difluoro (bisoxalato)phosphate, lithium difluorophosphate, tris(trimethylsilyl) phosphate, tris(2,2,2-trifluoroethyl)phosphate, or tris(trifluoroethyl)phosphate.

The borate-based compound may include tetraphenylborate or lithium oxalyldifluoroborate.

The nitrile-based compound may include at least one compound selected from succinonitrile, adiponitrile, acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, or 4-fluorophenylacetonitrile.

The benzene-based compound may include fluorobenzene, and the amine-based compound may include triethanolamine or ethylenediamine, and the like.

The silane-based compound may include tetravinylsilane.

The lithium salt-based compound is a compound different from the lithium salt included in the electrolyte, and may include one or more compounds selected from LiPO₂F₂, LiODFB, LiBOB(lithium bis (oxalato) borate (LiB(C₂O₄)₂), or LiBF₄.

In a case, in which at least one of vinylene carbonate, vinyl ethylene carbonate, succinonitrile or 1,3-propane sultone, among additional additives, is included, a more robust SEI film may be formed on the surface of the negative electrode during the initial activation process of the secondary battery.

The additional additives may be used as a mixture of two or more thereof, and may be present in an amount of 50 wt% or less, specifically 0.01 wt% to 10 wt%, and preferably 0.05 wt% to 5.0 wt% based on the total weight of the electrolyte. If the content of the additional additive is less than 0.01 wt%, the effects of improving low-temperature output, high-temperature storage characteristics, and high-temperature life characteristics of the battery are insignificant, and if the content of the additional additive is greater than 50 wt%, there is a possibility that side reactions may occur excessively in the electrolyte during the charge and discharge of the battery. Particularly, if the additives for forming an SEI film are added in excess, the additives may not be sufficiently decomposed at high temperatures so that they may be present in the form of unreacted materials or may precipitate in the electrolyte at room temperature. Accordingly, side reactions that degrade life or resistance characteristics of the secondary battery may occur.

### Lithium secondary battery

Further, in another embodiment of the present disclosure, the present disclosure provides a lithium secondary battery comprising the non-aqueous electrolyte for a lithium secondary battery of the present disclosure.

After an electrode assembly, in which the positive electrode, the negative electrode facing the positive electrode, and separator interposed between the positive electrode and the negative electrode are included, is accommodated in a battery case, the lithium secondary battery may be prepared by injecting the non-aqueous electrolyte thereto.

Since the non-aqueous electrolyte has been described above, the negative electrode, positive electrode, and separator will be described below.

### (1) Positive Electrode

The positive electrode may include a positive electrode active material.

The positive electrode active material is a compound capable of reversibly intercalation and deintercalation, is not particularly limited as long as it is a positive electrode active material used in the art, and specifically may include a lithium metal composite oxide. More specifically, the lithium metal composite oxide may include a lithium composite metal oxide represented by Formula 2 below, wherein the lithium composite metal oxide includes lithium and at least one metal selected from nickel (Ni), cobalt (Co), manganese (Mn), iron (Fe) or aluminum (Al).

[Formula 2] Li₁₊ₐNiₓCo_{y}M¹_{z}M²_{w}O₂

In Formula 2,
M¹ is Mn, Al or a combination thereof,
M² is at least one selected from Al, Zr, W, Ti, Mg, Ca or Sr, wherein 0≤a≤0.5, 0.55<x<1.0, 0<y≤0.4, 0<z≤0.4, 0≤w≤0.1.
1+a represents a molar ratio of lithium in a lithium transition metal oxide, and may be 0≤a≤0.5, preferably 0≤a≤0.2, and more preferably 0≤a≤0.1.
x represents a molar ratio of nickel among all transition metal elements except for lithium in the lithium transition metal oxide, and may be 0.55<x<1.0, specifically 0.6≤x≤0.98, and more specifically 0.6≤x≤0.95.
y represents a molar ratio of cobalt among all transition metal elements except for lithium in the lithium transition metal oxide, and may be 0<y≤0.4, specifically 0<y≤0.3, and more specifically 0.05≤y≤0.3.
z represents a molar ratio of the element M¹ among all transition metal elements except for lithium in the lithium transition metal oxide, and may be 0<z≤0.4, more specifically 0<z≤0.3, and even more specifically 0.01≤z≤0.3.
w represents a molar ratio of the element M² among all transition metal elements except for lithium in the lithium transition metal oxide, and may be 0<w≤0.1, more specifically 0<w≤0.05, and even more specifically 0<w≤0.02.

Specifically, the positive electrode active material may include a lithium composite transition metal oxide such as Li(Ni_{0.6}Mn_{0.2}Co_{0.2})O₂, Li(Ni_{0.7}Mn_{0.15}Co_{0.15})O₂, Li(Ni_{0.7}Mn_{0.2}Co_{0.1})O₂, Li(Ni_{0.8}Mn_{0.1}Co_{0.1})O₂, Li(Ni_{0.8}Co_{0.15}Al_{0.05})O₂, Li(Ni_{0.86}Mn_{0.07}Co_{0.05}Al_{0.02})O₂ or Li(Ni_{0.90}Mn_{0.05}Co_{0.05})O₂ having a Ni content of 0.55 atm% or more to achieve a high capacity battery, and specifically may include a high-Ni based lithium composite transition metal oxide such as Li(Ni_{0.8}Mn_{0.1}Co_{0.1})O₂, Li(Ni_{0.8}Co_{0.15}Al_{0.05})O₂, Li(Ni_{0.86}Mn_{0.07}Co_{0.05}Al_{0.02})O₂ or Li(Ni_{0.90}Mn_{0.05}Co_{0.05})O₂, etc.

The positive electrode active material including such a high Ni-based lithium composite transition metal oxide has the disadvantage of being vulnerable to side reactions with the electrolyte, but when it is used together with the electrolyte of the present disclosure, a stable passivation film is formed on the positive electrode surface, thereby suppressing and reducing the side reactions with the electrolyte.

Further, as the positive electrode active material of the present disclosure, a lithium-manganese-based oxide (e.g., LiMnO₂, LiMn₂O₄, etc.), a lithium-cobalt-based oxide (e.g., LiCoO₂, etc.), a lithium-nickel-based oxide (e.g., LiNiO₂, etc.), a lithium-nickel-manganese-based oxide (e.g., LiNi_{1-Y}Mn_{Y}O₂(0<Y<1), LiMn_{2-z}Ni_{z}O₄(0 < Z < 2), a lithium-nickel-cobalt-based oxide (e.g. , LiNi_{1-Y1}Co_{Y1}O₂(0<Y1<1)), a lithium-manganese-cobalt-based oxide (e.g., LiCo_{1-Y2}Mn_{Y2}O₂(0<Y2<1), LiMn_{2-z1}Co_{z1}O₄(0 < Z1 < 2), or Li(Niₚ₁Co_{q1}Mnᵣ₂)O₄(0<p1<2, 0<q1<2, 0<r2<2, p1+q1+r2=2)), etc. may be used together with a lithium composite metal oxide represented by Formula 2 above, if necessary.

The positive electrode active material may be in a particle form. Specifically, the average particle diameter (D₅₀) of the positive electrode active material may be in the range of 1*µ*m to 30*µ*m.

The positive electrode may include a positive electrode current collector and a positive electrode mixture layer disposed on at least one surface of the positive electrode current collector. The positive electrode mixture layer may include the above-described positive electrode active material.

The positive electrode current collector generally has a thickness of 3 to 500 µm.

Microscopic irregularities may be formed on the surface of the positive electrode current collector to improve the adhesion of the positive electrode active material. For example, the positive electrode current collector may be used in various shapes such as a film, a sheet, a foil, a net, a porous body, a foam body, a non-woven fabric body, and the like.

The positive electrode mixture layer may be disposed on at least one surface of the positive electrode current collector. Specifically, the positive electrode mixture layer may be disposed on one or both surfaces of the positive electrode current collector.

The positive electrode active material may be present in an amount of 70 wt% to 99 wt%, preferably 80 wt% to 98 wt% in the positive electrode mixture layer in consideration of the sufficient capacity of the positive electrode active material.

The positive electrode mixture layer may further include a binder and/or a conductive material together with the above-described positive electrode active material.

The binder improves the adhesion between the positive electrode active material particles and the adhesion between the positive electrode active material and a current collector. As an example of the binder, any one of a fluorine resin-based binder including polyvinylidene fluoride (PVDF) or polytetrafluoroethylene (PTFE); a rubber-based binder including a styrene butadiene rubber (SBR), an acrylonitrile-butadiene rubber, or a styrene-isoprene rubber; a cellulose-based binder including carboxy methyl cellulose (CMC), starch, hydroxy propyl cellulose, or regenerated cellulose; a polyalcohol-based binder including polyvinyl alcohol; a polyolefin-based binder including polyethylene or polypropylene; a polyimide-based binder; a polyester-based binder; or a silane-based binder or a mixture of two or more thereof may be used.

The binder may be present in an amount of 0.1 wt% to 15 wt%, preferably 0.1 wt% to 10 wt% based on the total weight of the positive electrode mixture layer.

Next, the conductive material is used to provide conductivity to the electrode, wherein any conductive material may be used without particular limitation as long as it has suitable electron conductivity without causing adverse chemical changes in the battery. Specific examples may include carbon black such as carbon black, acetylene black (or DENKA BLACK^{®}), KETJEN BLACK^{®}, channel black, furnace black, lamp black, or thermal black; graphite powder such as natural graphite with a well-developed crystal structure, artificial graphite, or graphite; conductive fibers such as carbon fibers or metal fibers; conductive powder such as fluorocarbon powder, aluminum powder, or nickel powder; conductive whiskers such as zinc oxide whiskers or potassium titanate whiskers; conductive metal oxide such as titanium oxide; polyphenylene derivatives, etc. and any one thereof or a mixture of two or more thereof may be used.

The conductive material may be present in an amount of 0.1 wt% to 10 wt%, preferably, 0.1 wt% to 5 wt% based on the total weight of the positive electrode mixture layer.

The positive electrode of the present disclosure may be prepared by a method of preparing a positive electrode which is known in the art. For example, the positive electrode may be manufactured by coating the positive electrode current collector with a positive electrode slurry including a positive electrode active material and optionally a binder, a conductive material and a solvent for forming a positive electrode slurry, and then drying and rolling. Alternatively, after a film is prepared by mixing the positive electrode active material and optionally a binder, a conductive material, etc., the film is then laminated on the positive electrode current collector to prepare a positive electrode.

The solvent for forming a positive electrode slurry may include, for example, at least one selected from distilled water, N-methyl pyrrolidone, ethanol, methanol, or isopropyl alcohol, preferably N-methyl pyrrolidone, for the purpose of facilitating the dispersion of the negative electrode active material, the binder and/or the conductive material.

### (2) Negative Electrode

Next, a negative electrode will be described.

The negative electrode according to the present disclosure includes a negative electrode mixture layer including a negative electrode active material, and the negative electrode mixture layer may further include a conductive material and/or a binder, if necessary.

As the above negative electrode active material, a silicon-based negative electrode active material can be used alone.

The silicon-based negative electrode active material, for example, may include one or more selected from metallic silicon (Si), silicon oxide (SiOₓ,where 0<x≤2), silicon carbide (SiC), or a Si-Y alloy (where Y is an element selected from alkali metal, alkaline earth metal, a Group 13 element, a Group 14 element, transition metal, a rare earth element, or a combination thereof, and is not Si). The element Y may be selected from Mg, Ca, Sr, Ba, Ra, Sc, Y, Ti, Zr, Hf, Rf, V, Nb, Ta, Db (dubnium), Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ti, Ge, P, As, Sb, Bi, S, Se, Te, Po, or a combination thereof.

Since the silicon-based negative electrode active material has higher capacity characteristics than the carbon-based negative electrode active material, better capacity characteristics may be obtained. However, with respect to a negative electrode including the silicon-based negative electrode active material, it contains more oxygen-rich (O-rich) components in an SEI than a graphite negative electrode, and the SEI containing the O-rich components tends to be more easily decomposed when a Lewis acid, such as HF or PF₅, is present in the electrolyte. Thus, with respect to the negative electrode containing the silicon-based negative electrode active material, there is a need to suppress the formation of Lewis acids, such as HF and PF₅ in the electrolyte, or remove (or scavenge) the formed Lewis acids in order to stably maintain the SEI. Since the non-aqueous electrolyte according to the present disclosure includes an electrolyte additive capable of forming a stable film on the surface of the silicon-based negative electrode, it may effectively suppress SEI film decomposition when the negative electrode containing the silicon-based active material is used.

Meanwhile, the negative electrode may additionally include, in addition to the silicon-based negative electrode active material, a conventional negative electrode active material capable of reversibly intercalating/deintercalating lithium ions, and may include a carbon-based negative electrode active material as a specific example.

As the carbon-based negative electrode active material, various carbon-based negative electrode active materials used in the art, for example, a graphite-based materials such as natural graphite, artificial graphite, or Kish graphite; pyrolytic carbon, mesophase pitch based carbon fiber, meso-carbon microbeads, mesophase pitches, high-temperature sintered carbon such as petroleum or coal tar pitch derived cokes, soft carbon, or hard carbon may be used. A shape of the carbon-based negative electrode active material is not particularly limited, and materials of various shapes, such as an irregular shape, planar shape, flaky shape, spherical shape, or fibrous shape, may be used.

Specifically, as the carbon-based negative electrode active material, at least one carbon-based negative electrode active material of natural graphite or artificial graphite may be used. The natural graphite and the artificial graphite may be used together so that adhesion with the current collector may be increased to suppress exfoliation of the active material.

Meanwhile, when the silicon-based negative electrode active material and the carbon-based negative electrode active material are be mixed, the mixing ratio thereof may be 3:97 to 99:1, and preferably 5:95 to 15:85. In a case in which the mixing ratio of the silicon-based negative electrode active material to the carbon-based negative electrode active material satisfies the above range, since the volume expansion of the silicon-based negative electrode active material is suppressed while capacity characteristics are improved, excellent cycle performance may be secured.

The negative electrode may include a negative electrode current collector and a negative electrode mixture layer disposed on at least one surface of the negative electrode current collector. The negative electrode active material may be included in the negative electrode mixture layer.

The negative electrode current collector is not particularly limited so long as it has a high conductivity without causing adverse chemical changes in the battery. Specifically, copper, stainless steel, aluminum, nickel, titanium, fired carbon, copper or stainless steel that is surface-treated with one of carbon, nickel, titanium, silver, and the like, and an aluminum-cadmium alloy may be used as the negative electrode current collector.

The negative electrode current collector generally has a thickness of 3 to 500 µm.

Microscopic irregularities may be formed on the surface of the negative electrode collector to improve the adhesion of the negative electrode active material. For example, the negative electrode current collector may be used in various shapes such as a film, a sheet, a foil, a net, a porous body, a foam body, a non-woven fabric body, and the like.

The negative electrode mixture layer may be disposed on at least one surface of the negative electrode current collector. Specifically, the negative electrode mixture layer may be disposed on one or both surfaces of the negative electrode current collector.

The negative electrode active material may be present in an amount of 60 wt% to 99 wt% in the negative electrode mixture layer in terms of exhibiting the sufficient capacity in the secondary battery while minimizing the impact of the volume expansion/contraction on the battery. In a case in which the amount of the negative electrode active material satisfies the above range, excellent capacity characteristics and electrochemical properties may be obtained.

The negative electrode mixture layer may further include a conductive material and/or a binder together with the above silicon-based active material.

The conductive material is a component for further improving conductivity of the negative electrode active material, wherein the conductive material may be added in an amount of 10 wt% or less, preferably 5 wt% or less based on the total weight of the negative electrode mixture layer. Any conductive material may be used without particular limitation so long as it has conductivity without causing adverse chemical changes in the battery, and, for example, carbon black such as carbon black, acetylene black (or DENKA BLACK^{®})), KETJEN BLACK^{®}, channel black, furnace black, lamp black, or thermal black; graphite powder such as natural graphite with a well-developed crystal structure, artificial graphite, or graphite; conductive fibers such as carbon fibers or metal fibers; conductive powder such as fluorocarbon powder, aluminum powder, or nickel powder; conductive whiskers such as zinc oxide whiskers or potassium titanate whiskers; conductive metal oxide such as titanium oxide; or polyphenylene derivatives, may be used.

The binder is a component that assists in the binding between the conductive material, the active material, and the current collector, wherein the binder is commonly added in an amount of 0.1 wt% to 10 wt% based on the total weight of the negative electrode mixture layer. Examples of the binder may be a fluorine resin-based binder including polyvinylidene fluoride (PVDF) or polytetrafluoroethylene (PTFE); a rubber-based binder including a styrene butadiene rubber (SBR), an acrylonitrile-butadiene rubber, or a styrene-isoprene rubber; a cellulose-based binder including carboxy methyl cellulose (CMC), starch, hydroxy propyl cellulose, or regenerated cellulose; a polyalcohol-based binder including polyvinyl alcohol; a polyolefin-based binder including polyethylene or polypropylene; a polyimide-based binder; a polyester-based binder; or a silane-based binder.

The binder may be present in an amount of 0.1 wt% to 15 wt%, preferably 0.1 wt% to 10 wt% based on the total weight of the negative electrode mixture layer.

The negative electrode may be prepared by a method of preparing a negative electrode which is known in the art. For example, the negative electrode may be manufactured by coating the negative electrode current collector with a negative electrode slurry including a negative electrode active material and optionally a binder, a conductive material and a solvent for forming a negative electrode slurry, and then drying and rolling. Alternatively, after a film is prepared by mixing the negative electrode active material and optionally a binder, a conductive material, etc., the film is then laminated on the negative electrode current collector to prepare a negative electrode.

The solvent for forming a negative electrode slurry may include, for example, at least one selected from distilled water, N-methyl pyrrolidone, ethanol, methanol, or isopropyl alcohol, and preferably distilled water, for the purpose of facilitating the dispersion of the negative electrode active material, the binder and/or the conductive material.

### (3) Separator

The lithium secondary battery according to the present disclosure includes a separator between the positive electrode and the negative electrode.

The separator separates the negative electrode and the positive electrode and provides a movement path of lithium ions, wherein any separator may be used as the separator without particular limitation as long as it is typically used in a lithium secondary battery, and particularly, a separator having high moisture-retention ability for an electrolyte as well as low resistance to the transfer of lithium salt ions is preferable.

Specifically, a porous polymer film, for example, a porous polymer film prepared from a polyolefin-based polymer, such as an ethylene homopolymer, a propylene homopolymer, an ethylene/butene copolymer, an ethylene/hexene copolymer, or an ethylene/methacrylate copolymer, or a laminated structure having two or more layers thereof may be used as the separator. Also, a typical porous nonwoven fabric, for example, a nonwoven fabric formed of high melting point glass fibers or polyethylene terephthalate fibers may be used. Furthermore, a coated separator including a ceramic component or a polymer material may be used to secure heat resistance or mechanical strength, and the separator having a single layer or multilayer structure may be optionally used.

The lithium secondary battery according to the present disclosure as described above may be suitably used in portable devices, such as mobile phones, notebook computers, and digital cameras, and electric cars such as hybrid electric vehicles (HEVs).

A shape of the lithium secondary battery of the present disclosure is not particularly limited, but a cylindrical type using a can, a prismatic type, a pouch type, or a coin type may be used.

The lithium secondary battery according to the present disclosure may not only be used in a battery cell that is used as a power source of a small device, but may also be used as a unit cell in a medium and large sized battery module including a plurality of battery cells.

Hereinafter, the present disclosure will be described in more detail according to examples. However, the invention may be embodied in many different forms and should not be construed as being limited to the embodiments set forth herein. Rather, these example embodiments are provided so that this description will be thorough and complete, and will fully convey the scope of the present disclosure to those skilled in the art.

### Examples

### Example 1

### (Preparation of non-aqueous electrolyte for a lithium secondary battery)

LiPF₆ was dissolved in a non-aqueous organic solvent, in which ethylene carbonate (EC) and ethylmethyl carbonate (EMC) were mixed in a volume ratio of 30:70 such that a concentration of the LiPF₆ was 1.0M, and a non-aqueous electrolyte for a lithium secondary battery was then prepared by adding 0.1 wt% of a compound represented by Formula 1A-1, 0.5 wt% of vinylene carbonate (VC), and 0.5 wt% of 1,3-propane sultone (PS) (see Table 1 below).

### (Preparation of Secondary Battery)

A positive electrode active material particle (lithium nickel-manganese-cobalt-aluminum oxide; Li(Ni_{0.86}Mn_{0.07}Co_{0.05}Al_{0.02})O₂) : a conductive material (carbon black): a binder (polyvinylidene fluoride) were added in a weight ratio of 97.74:0.7:1.56 to N-methyl-2-pyrrolidone (NMP), which was a solvent, to prepare a positive electrode slurry (solid content 75.50 wt%). A 15 µm thick positive electrode collector (Al thin film) was coated with the positive electrode slurry, dried, and roll-pressed to prepare a positive electrode.

A negative electrode active material (graphite: SiO=95:5 weight ratio), a conductive material (carbon black), and a binder (SBR-CMC) were added to water, which is a solvent, at a weight ratio of 97.8 : 0.2 : 2.0 to prepare a negative electrode slurry (solid content: 26 wt%). A 15*µ*m thick negative electrode collector (Cu thin film) was coated with the negative electrode slurry, dried, and roll-pressed to prepare a negative electrode.

After an electrode assembly was prepared by disposing the porous polypropylene separator between the positive electrode and the negative electrode, the electrode assembly was accommodated in a battery case, and the above prepared non-aqueous electrolyte for a lithium secondary battery was injected thereinto to prepare a lithium secondary battery.

### Example 2

### (Preparation of non-aqueous electrolyte for a lithium secondary battery)

A non-aqueous electrolyte for a lithium secondary battery was prepared in the same manner as in Example 1 except that LiPF₆ was dissolved in an organic solvent such that a concentration of the LiPF₆ was 1.0 M, and 0.5 wt% of a compound represented by Formula 1A-1, 0.5 wt% of vinylene carbonate (VC), and 0.5 wt% of 1,3-propane sultone (PS) were added (see Table 1 below).

### (Preparation of Secondary Battery)

A lithium secondary battery was prepared in the same manner as in Example 1 except that the above prepared non-aqueous electrolyte for a lithium secondary battery was injected.

### Example 3

### (Preparation of non-aqueous electrolyte for a lithium secondary battery)

A non-aqueous electrolyte for a lithium secondary battery was prepared in the same manner as in Example 1 except that LiPF₆ was dissolved in an organic solvent such that a concentration of the LiPF₆ was 1.0 M, and 1.0 wt% of a compound represented by Formula 1A-1, 0.5 wt% of vinylene carbonate (VC), and 0.5 wt% of 1,3-propane sultone (PS) were added (see Table 1 below).

### (Preparation of Secondary Battery)

A lithium secondary battery was prepared in the same manner as in Example 1 except that the above prepared non-aqueous electrolyte for a lithium secondary battery was injected.

### Example 4

### (Preparation of non-aqueous electrolyte for a lithium secondary battery)

A non-aqueous electrolyte for a lithium secondary battery was prepared in the same manner as in Example 1 except that LiPF₆ was dissolved in an organic solvent such that a concentration of the LiPF₆ was 1.0 M, and 3.0 wt% of a compound represented by Formula 1A-1, 0.5 wt% of vinylene carbonate (VC), and 0.5 wt% of 1,3-propane sultone (PS) were added (see Table 1 below).

### (Preparation of Secondary Battery)

A lithium secondary battery was prepared in the same manner as in Example 1 except that the above prepared non-aqueous electrolyte for a lithium secondary battery was injected.

### Example 5

### (Preparation of non-aqueous electrolyte for a lithium secondary battery)

A non-aqueous electrolyte for a lithium secondary battery was prepared in the same manner as in Example 1 except that LiPF₆ was dissolved in an organic solvent such that a concentration of the LiPF₆ was 1.0M, and 5.0 wt% of a compound represented by Formula 1A-1, 0.5 wt% of vinylene carbonate (VC), and 0.5 wt% of 1,3-propane sultone (PS) were added (see Table 1 below).

### (Preparation of Secondary Battery)

A lithium secondary battery was prepared in the same manner as in Example 1 except that the above prepared non-aqueous electrolyte for a lithium secondary battery was injected.

### Example 6

### (Preparation of non-aqueous electrolyte for a lithium secondary battery)

A non-aqueous electrolyte for a lithium secondary battery was prepared in the same manner as in Example 3 except that it was prepared by adding a compound represented by Formula 1A-2 instead of a compound represented by Formula 1A-1 (see Table 1 below).

### (Preparation of Secondary Battery)

A lithium secondary battery was prepared in the same manner as in Example 1 except that the above prepared non-aqueous electrolyte for a lithium secondary battery was injected.

### Example 7

### (Preparation of non-aqueous electrolyte for a lithium secondary battery)

A non-aqueous electrolyte for a lithium secondary battery was prepared in the same manner as in Example 3 except that it was prepared by adding a compound represented by Formula 1A-3 instead of a compound represented by Formula 1A-1 (see Table 1 below).

### (Preparation of Secondary Battery)

A lithium secondary battery was prepared in the same manner as in Example 1 except that the above prepared non-aqueous electrolyte for a lithium secondary battery was injected.

### Example 8

### (Preparation of non-aqueous electrolyte for a lithium secondary battery)

A non-aqueous electrolyte for a lithium secondary battery was prepared in the same manner as in Example 1 except that LiPF₆ was dissolved in an organic solvent such that a concentration of the LiPF₆ was 1.0M, and 7.0 wt% of a compound represented by Formula 1A-1, 0.5 wt% of vinylene carbonate (VC), and 0.5 wt% of 1,3-propane sultone (PS) were added (see Table 1 below) .

### (Preparation of Secondary Battery)

A lithium secondary battery was prepared in the same manner as in Example 1 except that the above prepared non-aqueous electrolyte for a lithium secondary battery was injected.

### Example 9

### (Preparation of non-aqueous electrolyte for a lithium secondary battery)

A non-aqueous electrolyte for a lithium secondary battery was prepared in the same manner as in Example 1 except that LiPF₆ was dissolved in an organic solvent such that a concentration of the LiPF₆ was 1.0M, and 10.0 wt% of a compound represented by Formula 1A-1, 0.5 wt% of vinylene carbonate (VC), and 0.5 wt% of 1,3-propane sultone (PS) were added (see Table 1 below).

### (Preparation of Secondary Battery)

A lithium secondary battery was prepared in the same manner as in Example 1 except that the above prepared non-aqueous electrolyte for a lithium secondary battery was injected.

### Comparative Example 1

### (Preparation of non-aqueous electrolyte for a lithium secondary battery)

A non-aqueous electrolyte for a lithium secondary battery was prepared in the same manner as in Example 1 except that LiPF₆ was dissolved in a non-aqueous organic solvent such that a concentration of the LiPF₆ was 1.5 M, and 0.5 wt% of vinylene carbonate (VC) and 0.5 wt% of 1,3-propane sultone (PS) were added as additives (as see Table 1 below).

### (Preparation of Secondary Battery)

A lithium secondary battery was prepared in the same manner as in Example 1 except that the above prepared non-aqueous electrolyte for a lithium secondary battery was injected.

### Comparative Example 2

### (Preparation of non-aqueous electrolyte for a lithium secondary battery)

A non-aqueous electrolyte for a lithium secondary battery was prepared in the same manner as in Example 3 except that it was prepared by adding 1.0 wt% of a compound represented by Formula 3 below instead of a compound represented by Formula 1A-1 (see Table 1 below).

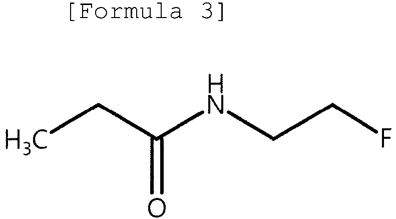

### (Preparation of Lithium Secondary Battery)

A lithium secondary battery was prepared in the same manner as in Example 1 except that the above prepared non-aqueous electrolyte for a lithium secondary battery was injected.

**[Table 1]**

| | Non-aqueous organic solvent | Additive | | Additional additive | |
|---|---|---|---|---|---|
| | | Types | Content (wt%) | Types | Content (wt%) |
| Example 1. | EC:EMC=30: 70 volume ratio | [Formula 1A-1] | 0.1 | VC/PS | 0.5/ 0.5 |
| Example 2 | | [Formula 1A-1] | 0.5 | | |
| Example 3 | | [Formula 1A-1] | 1.0 | | |
| Example 4 | | [Formula 1A-1] | 3.0 | | |
| Example 5 | | [Formula 1A-1] | 5.0 | | |
| Example 6 | | [Formula 1A-2] | 1.0 | | |
| Example 7 | | [Formula 1A-3] | 1.0 | | |
| Example 8 | | [Formula 1A-1] | 7.0 | | |
| Example 9 | | [Formula 1A-1] | 10.0 | | |
| Comparative Example 1 | | - | - | | |
| Comparative Example 2 | | Formula 3 | 1.0 | | |

Meanwhile, the abbreviation of each compound has the following meaning in Table 1.
EC: Ethylene carbonate
EMC: Ethyl methyl carbonate
VC: Vinylene carbonate
PS: 1,3-propane sultone

### Experimental Examples

### Experimental Example 1 Evaluation of high-temperature cycle characteristics

Each of the secondary batteries prepared in Examples 1 to 9 and Comparative Examples 1 and 2 was evaluated for their high-temperature cycle characteristics.

Specifically, the secondary batteries prepared in Examples 1 to 9 and Comparative Examples 1 and 2 were charged to 4.2V at a constant-current of 1C at 45°C under a CC condition, and were then discharged to 2.5V at a constant-current of 1.0C under a CC condition, which was set as one cycle. After 200 cycles of charging and discharging were performed, the capacity retention rate compared to the initial capacity after 1 cycle was measured. The results are presented in Table 2 below.

**[Table 2]**

| | Capacity retention rate (%) after 200 cycles |
|---|---|
| Example 1 | 89.5 |
| Example 2 | 91.7 |
| Example 3 | 93.5 |
| Example 4 | 94.8 |
| Example 5 | 94.3 |
| Example 6 | 93.4 |
| Example 7 | 93.8 |
| Example 8 | 90.6 |
| Example 9 | 89.8 |
| Comparative Example 1 | 88.2 |
| Comparative Example 2 | 88.7 |

Referring to Table 2, it can be understood that the secondary batteries of Examples 1 to 9, where the electrolyte for a lithium secondary battery including the additive of the present disclosure, was used, exhibited relatively improved capacity retention rates after 200 cycles at high temperatures compared to the secondary batteries of Comparative Examples 1 and 2.

### Experimental Example 2 Evaluation of high-temperature storage characteristics

Each of the secondary batteries prepared in Examples 1 to 9 and Comparative Examples 1 and 2 was evaluated for their high-temperature storage characteristics.

Specifically, after the secondary batteries prepared in Examples 1 to 9 and the secondary batteries prepared in Comparative Examples 1 and 2 were fully charged to 4.3 V, they were stored at 60°C for 8 weeks.

Before storage, the capacity of the fully charged secondary battery was measured and set as the initial capacity of the secondary battery.

After 8 weeks, the capacity of the stored secondary batteries was measured, and the reduced capacity during the 8-week storage period was calculated. The ratio of the reduced capacity to the initial capacity of the secondary battery was calculated as a percentage to derive the capacity retention rate after 8 weeks. The results are presented in Table 3 below.

**[Table 3]**

| | Capacity retention rate (%) after high-temperature storage for 8 weeks |
|---|---|
| Example 1 | 87.1 |
| Example 2 | 91.3 |
| Example 3 | 92.5 |
| Example 4 | 93.5 |
| Example 5 | 94.1 |
| Example 6 | 92.2 |
| Example 7 | 92.6 |
| Example 8 | 92.0 |
| Example 9 | 91.1 |
| Comparative Example 1 | 86.2 |
| Comparative Example 2 | 86.7 |

Referring to Table 3, it can be understood that the secondary batteries of Examples 1 to 9, where the electrolyte for a lithium secondary battery including the additive of the present disclosure, have improved capacity retention rates after high-temperature storage compared to the secondary batteries of Comparative Examples 1 and 2.

## Claims

1. An electrolyte additive including a compound represented by Formula 1: 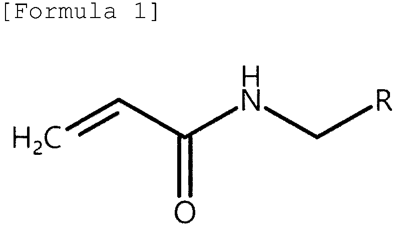
wherein, in Formula 1,
R is an alkyl group having 1 to 7 carbon atoms substituted with at least one fluorine.

2. The electrolyte additive according to claim 1, wherein, in Formula 1, R is an alkyl group having 1 to 5 carbon atoms substituted with at least one fluorine.

3. The electrolyte additive according to claim 1, wherein, in Formula 1, R is an alkyl group having 1 to 3 carbon atoms substituted with at least one fluorine.

4. The electrolyte additive according to claim 1, wherein the compound represented by Formula 1 is at least one of compounds represented by Formulas 1A-1 to 1A-3:

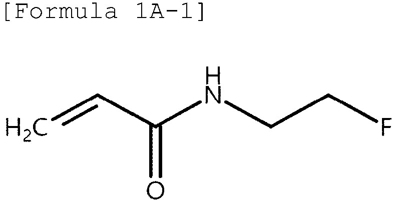

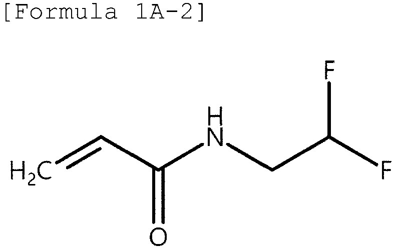

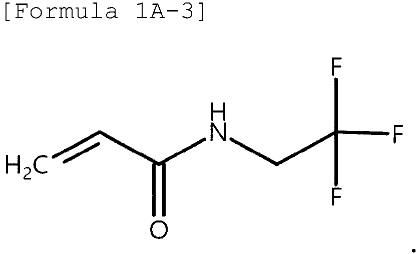

5. A non-aqueous electrolyte for a lithium secondary battery comprising the electrolyte additive of claim 1

6. The non-aqueous electrolyte for a lithium secondary battery according to claim 5, wherein the electrolyte additive is present in an amount of 0.1 wt% to 10.0 wt% in the non-aqueous electrolyte for a lithium secondary battery.

7. The non-aqueous electrolyte for a lithium secondary battery according to claim 5, wherein the non-aqueous electrolyte for a lithium secondary battery further includes a lithium salt and an non-aqueous organic solvent.

8. The non-aqueous electrolyte for a lithium secondary battery according to claim 5, wherein the non-aqueous electrolyte for a lithium secondary battery further includes at least one additional additive selected from a group consisting of a cyclic carbonate-based compound, a halogen-substituted carbonate-based compound, a sultone-based compound, a sulfate-based compound, a phosphate-based or phosphite-based compound, a borate-based compound, a benzene-based compound, an amine-based compound, an imidazole-based compound, a silane-based compound, and a lithium salt-based compound.

9. A lithium secondary battery comprising a positive electrode;
a negative electrode facing the positive electrode;
a separator disposed between the positive electrode and the negative electrode, and
the non-aqueous electrolyte for a lithium secondary battery according to claim 5.

10. The lithium secondary battery according to claim 9, wherein the negative electrode includes a silicon-based negative electrode active material.

11. The lithium secondary battery according to claim 10, wherein the negative electrode further includes a carbon-based negative electrode active material.
